(19) Europäisches Patentamt
European Patent Office
Office européen des brevets

(11) **EP 4 475 132 A1**

(12) **EUROPEAN PATENT APPLICATION**
published in accordance with Art. 153(4) EPC

(43) Date of publication:
**11.12.2024 Bulletin 2024/50**

(21) Application number: 23749490.1

(22) Date of filing: **16.01.2023**

(51) International Patent Classification (IPC):
**G16C 60/00** (2019.01)

(52) Cooperative Patent Classification (CPC):
**G06Q 50/10; G16C 60/00**

(86) International application number:
**PCT/JP2023/000900**

(87) International publication number:
**WO 2023/149173 (10.08.2023 Gazette 2023/32)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **01.02.2022 JP 2022014402**

(71) Applicant: **Sumitomo Chemical Company, Limited
Tokyo 103-6020 (JP)**

(72) Inventors:
• **KIKUCHI, Yuta
Ichihara-shi, Chiba 299-0195 (JP)**
• **TAKAGI, Wataru
Tsukuba-shi, Ibaraki 300-3294 (JP)**

(74) Representative: **Vossius & Partner
Patentanwälte Rechtsanwälte mbB
Siebertstrasse 3
81675 München (DE)**

(54) **INFORMATION PROCESSING METHOD, PROGRAM, INFORMATION PROCESSING DEVICE, METHOD FOR GENERATING MODEL, METHOD FOR PRODUCING METAL ORGANIC STRUCTURE, METAL ORGANIC STRUCTURE, AND COMPOSITE**

(57) Provided are an information processing method and the like, capable of efficiently estimating the adsorption-desorption characteristic of a metal organic framework.

An information processing method, includes: acquiring a plurality of parameters in a metal organic framework; and outputting an adsorption-desorption characteristic by inputting the plurality of acquired parameters to a first model trained to output an adsorption-desorption characteristic relevant to adsorption or desorption in a metal organic framework when a plurality of parameters in the metal organic framework are input.

FIG.1

EP 4 475 132 A1

## Description

Technical Field

The present invention relates to an information processing method, a program, an information processing device, a method for creating a model, a method for producing a metal organic framework, a metal organic framework, and a complex.

Background Art

A metal organic framework (MOF, hereinafter, also referred to as MOF), which is a porous compound, is a material to be also referred to as a porous coordination polymer (PCP). MOF has a coordination network structure with a high surface area, which is formed by an interaction between a metal and an organic ligand.

MOF exhibits a characteristic of adsorbing or desorbing water or gas, organic molecules, or the like by the structure described above, and thus, can be used as various materials. Therefore, research and development relevant to MOF has progressed. In Patent Literature 1, a method for producing a metal organic framework having a high moisture-absorption performance even in a low humidity condition is proposed.

Citation List

Patent Literature:

Patent Literature 1: Japanese Patent Laid-Open Publication No. 2020-11943 Summary

Technical Problems

In the case of producing MOF exhibiting high required performance, it is necessary to select MOF satisfying a characteristic according to the purpose of use by evaluating the characteristic of each MOF among a plurality of MOFs, and determine MOF to be a production target. It takes time and cost to individually evaluate the characteristic in the plurality of MOFs.

An object of the present disclosure is to provide an information processing method and the like, capable of efficiently estimating the adsorption-desorption characteristic of MOF.

Solution to Problems

An information processing method according to one aspect of the present disclosure, includes: acquiring a plurality of parameters in a metal organic framework; and outputting an adsorption-desorption characteristic by inputting the plurality of acquired parameters to a first model trained to output an adsorption-desorption characteristic relevant to adsorption or desorption in a metal organic framework when a plurality of parameters in the metal organic framework are input.

A method for creating a model according to one aspect of the present disclosure, includes: acquiring training data including a plurality of parameters in a metal organic framework, and an adsorption-desorption characteristic relevant to adsorption or desorption in the metal organic framework; and creating a model trained to output an adsorption-desorption characteristic relevant to adsorption or desorption in a metal organic framework when a plurality of parameters in the metal organic framework are input, on the basis of the training data.

A method for producing a metal organic framework according to one aspect of the present disclosure, includes: a step of acquiring a plurality of parameters in a metal organic framework to be a candidate; a step of outputting an adsorption-desorption characteristic by inputting the plurality of acquired parameters to a first model trained to output an adsorption-desorption characteristic relevant to adsorption or desorption in a metal organic framework when a plurality of parameters in the metal organic framework are input; a step of specifying a metal organic framework of which the output adsorption-desorption characteristic satisfies a predetermined condition; and a step of obtaining the specified metal organic framework.

A metal organic framework according to one aspect of the present disclosure is obtained by the information processing method described above.

A complex according to one aspect of the present disclosure contains a metal organic framework obtained by the information processing method described above.

Advantageous Effects of Invention

According to the present disclosure, it is possible to efficiently estimate the adsorption-desorption characteristic of MOF.

Brief Description of Drawings

FIG. 1 is a block diagram illustrating a configuration of an information processing device according to an embodiment.
FIG. 2 is an explanatory diagram illustrating an outline of a first model.
FIG. 3 is an explanatory diagram illustrating an outline of a second model.
FIG. 4 is a diagram illustrating a content example of MOF data.
FIG. 5 is a diagram illustrating an example of a heat map indicating a correlative relationship.
FIG. 6 is a diagram illustrating an example of a heat map indicating an extraction result.
FIG. 7 is a flowchart illustrating an example of a processing procedure relevant to primary extraction.

FIG. 8 is a flowchart illustrating an example of a processing procedure relevant to secondary extraction.

FIG. 9 is a flowchart illustrating an example of a processing procedure relevant to tertiary extraction.

Description of Embodiments

The present disclosure will be described in detail with reference to the drawings representing embodiments thereof. First, an information processing method using an information processing device according to an embodiment will be described.

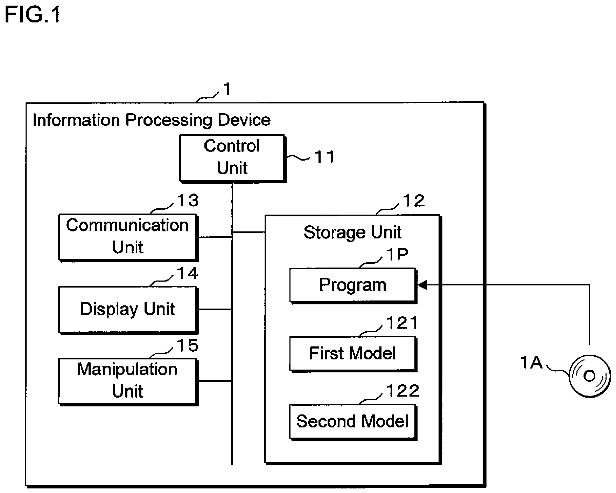

FIG. 1 is a block diagram illustrating the configuration of an information processing device 1 according to an embodiment. The information processing device 1 is a device capable of performing various information processing pieces, and transmission and reception of information, and for example, is a server computer, a personal computer, a quantum computer, or the like. The information processing device 1 estimates an adsorption-desorption characteristic in a plurality of MOFs using a model described below. The adsorption-desorption characteristic is a characteristic relevant to the adsorption or the desorption (the elimination) of MOF. The information processing device 1 extracts MOF satisfying a predetermined adsorption-desorption characteristic, on the basis of the estimated adsorption-desorption characteristic. Accordingly, it is possible to efficiently select the candidates of MOF to be a production (preparation) target among the plurality of MOFs.

In this embodiment, as an example, a case of extracting MOF specialized in the adsorption of water will be described, but the adsorption-desorption characteristic required for MOF to be an extraction target is not limited to the adsorption of water, and for example, may be a performance relevant to the desorption of water, the adsorption or the desorption of gas, the adsorption or the desorption of organic molecules, or the like. Examples of the gas include carbon dioxide, hydrogen, carbon monoxide, oxygen, nitrogen, hydrocarbon having 1 to 4 carbon atoms, rare gas, hydrogen sulfide, ammonia, a sulfur oxide, a nitrogen oxide, and siloxane, and examples of the organic molecules include hydrocarbon having 5 to 8 carbon atoms, alcohol having 1 to 8 carbon atoms, aldehyde having 1 to 8 carbon atoms, a carboxylic acid having 1 to 8 carbon atoms, ketone having 1 to 8 carbon atoms, amine having 1 to 8 carbon atoms, ester having 1 to 8 carbon atoms, and amide having 1 to 8 carbon atoms. Note that, the organic molecules may contain an aromatic ring.

The information processing device 1 includes a control unit 11, a storage unit 12, a communication unit 13, a display unit 14, a manipulation unit 15, and the like. The information processing device 1 may be a multicomputer consisting of a plurality of computers, or may be a virtual machine virtually constructed by software.

The control unit 11 is an arithmetic processing device including a central processing unit (CPU), a graphics processing unit (GPU), and the like. The control unit 11 controls each configuration unit and executes processing using a built-in memory, such as a read only memory (ROM) or a random access memory (RAM), clock, counter, or the like.

The storage unit 12 includes a non-volatile storage device such as a hard disk or a solid state drive (SSD). The storage unit 12 stores a program and data to which the control unit 11 refers. The storage unit 12 may be composed of a plurality of storage devices, or may be an external storage device connected to the information processing device 1. A computer program stored in the storage unit 12 includes a program 1P for causing a computer to execute processing relevant to the estimation or the extraction of the adsorption-desorption characteristic of MOF.

The computer program (a computer program product) stored in the storage unit 12 may be provided by a non-transitory recording medium 1A in which the computer program is recorded to be readable. The recording medium 1A is a portable memory such as a CD-ROM, a USB memory, and a secure digital (SD) card. The control unit 11 reads out a desired computer program from the recording medium 1A using a reading device that is not illustrated, and stores the read computer program in the storage unit 12. Alternatively, the computer program may be provided by communication. The program 1P can be disposed on a single computer or one site, or can be dispersed over a plurality of sites, and decompressed to be executed on a plurality of computers connected to each other by a communication network.

The data stored in the storage unit 12 includes a first model 121 for estimating the adsorption-desorption characteristic of MOF and a second model 122 for estimating the parameter of MOF. Each of the first model 121 and the second model 122 is a machine learning model created by machine learning. The first model 121 and the second model 122 are assumed to be used as a program module configuring a part of artificial intelligence software.

The communication unit 13 includes a communication device for performing processing relevant to communication through a network such as the Internet. The control unit 11 performs the transmission and reception of various information pieces with respect to an external device through the communication unit 13.

The display unit 14 includes a display device such as a liquid crystal panel and an organic electro luminescence (EL) display. The display unit 14 displays various information pieces, according to an instruction from the control unit 11.

The manipulation unit 15 is an interface for receiving the manipulation of a user, and for example, includes such as a keyboard, a touch panel device with a built-in display, a speaker, a microphone, and the like. The manipulation unit 15 receives the manipulation input from the user, and transmits a control signal according to manipulation contents to the control unit 11.

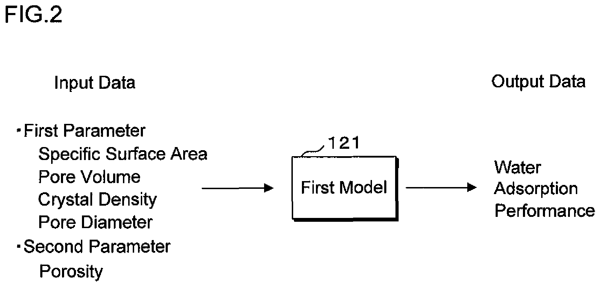

FIG. 2 is an explanatory diagram illustrating the outline of the first model 121. The first model 121 is a model that has learned predetermined training data. The first model 121 outputs the adsorption-desorption characteristic of the MOF using a plurality of parameters in MOF as input.

The parameters in MOF are parameters for defining MOF, and for example, include the physical property value of MOF, a value representing the structure of MOF, and the like. The parameters in MOF are sorted into a first parameter and a second parameter, according to the type of parameter. In the example illustrated in FIG. 2, parameters input to the first model 121 include four types of first parameters, and one type of second parameter. The first parameter is the specific surface area, the pore volume, the crystal density, and the pore diameter of MOF. The second parameter is a porosity. In the following description, in a case where it is not necessary to describe the first parameter and the second parameter by distinguishing the parameters from each other, the first parameter and the second parameter will also be simply referred to as the parameter. The details of each of the parameters will be described below.

The adsorption-desorption characteristic of MOF is to define a characteristic required for MOF to be the production target. In the example illustrated in FIG. 2, the adsorption-desorption characteristic output from the first model 121 is a water adsorption performance (a water adsorption amount) in MOF. The adsorption-desorption characteristic includes a first water adsorption performance that is a water adsorption performance at relative humidity of 10%, and a second water adsorption performance that is a water adsorption performance at relative humidity of 30%.

The first model 121, for example, is a multiple linear regression model. The first model 121 is a multiple linear regression formula using each of the parameters in MOF as an explanatory variable, and the adsorption-desorption characteristic as an object variable. The multiple linear regression formula, for example, can be represented as follows.

$$y = a_1x_1 + a_2x_2 + \ldots + a_nx_n + a_0$$

Here, $x_1$ to $x_n$ are each of the parameters, y is the adsorption-desorption characteristic, and $a_0$ to $a_n$ are coefficients.

The multiple linear regression formula may be derived with respect to each adsorption-desorption characteristic to be the object variable. For example, the first model 121 for outputting each of the first water adsorption performance and the second water adsorption performance may be constructed.

The information processing device 1 collects in advance an information group in which a known water adsorption performance is applied to the first parameter and the second parameter of MOF, as training data and learns the first model 121. Specifically, the information processing device 1 executes multiple linear regression analysis on the prepared training data by using each of the parameters as an explanatory variable, and the water adsorption performance as an object variable. The information processing device 1 derives a multiple linear regression formula using the value of the explanatory variable as an argument, and an output value as an object variable. The information processing device 1 learns the coefficients $a_0$ to $a_n$ for each of the data pieces such that the output value of y (the water adsorption performance) approaches the water adsorption performance that is a ground truth value.

The information processing device 1, as described above, derives the multiple linear regression formula with respect to each of the water adsorption-desorption characteristics. As described above, the first model 121 trained to be capable of suitably estimating the water adsorption performance with respect to the first parameter and the second parameter can be constructed.

The adsorption-desorption characteristic of MOF is affected by the structure of a metal and an organic ligand contained in MOF. For example, in a case where the specific surface area is large, the water adsorption amount may be large. For example, in a case where the porosity is large, the water adsorption amount may be large. The first model 121 according to such findings is created.

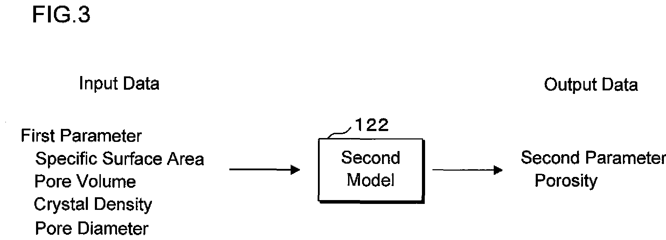

FIG. 3 is an explanatory diagram illustrating the outline of the second model 122. The second model 122 is a model that has learned predetermined training data. The second model 122 outputs the second parameter in MOF using a plurality of first parameters in MOF as input. The second parameter is a parameter with a type different from that of the first parameter.

The second model 122, for example, is a multiple linear regression model. The second model 122 is a multiple linear regression formula using each of the first parameters in MOF as an explanatory variable, and the second parameter as an object variable. Since the configuration of the second model 122 is the same as that of the first model 121, the detailed description thereof will be omitted.

As described above, the first parameter is the specific surface area, the pore volume, the crystal density, and the pore diameter of MOF, and the second parameter is the porosity. The first parameter is various parameters relevant to the structure of MOF, and is a measured value relevant to the pore of MOF or a parameter that can be calculated by a calculation formula on the basis of the measured value. The second parameter is various parameters relevant to the structure of MOF, and for example, is a parameter obtained by using predetermined analysis software for X-ray crystallography. That is, the first parameter is a parameter that is comparatively easy to measure or calculate, and the second parameter is a parameter comparatively difficult to measure or calculate.

The information processing device 1 collects in advance an information group in which a known second parameter is applied to the first parameter of MOF, as training data and learns the second model 122. Specifically, the information processing device 1 executes multiple linear regression analysis on the prepared training data by using each of the first parameters as an explanatory variable, and the second parameter as an object variable. The information processing device 1 derives a multiple linear regression formula using the value of the explanatory variable as an argument, and an output value as an object variable. The information processing device 1 learns the coefficients $a_0$ to $a_n$ such that for each of the data pieces such that the output value of y (the second parameter) approaches the second parameter that is a ground truth value.

As described above, the second model 122 trained to be capable of suitably estimating the second parameter with respect to the first parameter can be constructed. The information processing device 1 constructs the second model 122 by collecting the information of MOF of which the first parameter that is easy to obtain and the second parameter that is difficult to obtain are known.

In the related art, the porosity is calculated by analysis software using the specific surface area, the pore volume, the crystal density, the pore diameter, the X-ray crystallography result, and the like, which are calculated in advance. Accordingly, dedicated analysis software is required, and a burden for analysis processing is required. In this embodiment, by using the second model 122, it is possible to easily estimate the second parameter, on the basis of the first parameter of any MOF. By using the obtained first parameter and second parameter, it is possible for the information processing device 1 to accurately estimate the adsorption performance by the first model 121.

Note that, the first parameter, the second parameter, and the water adsorption-desorption characteristic are merely an example. Each of the values that can be the input and the output of the first model 121 and the second model 122 is not limited to the examples described above, and may be suitably selected according to MOF to be extracted. The first parameter, for example, may include the molecular weight, the formula weight (a molecular weight per unit lattice), the valence of the metal, the functional group of the organic ligand, and the like in MOF. The adsorption-desorption characteristic, for example, may include the desorption performance (the desorption amount) of water, the adsorption performance of various gases, the desorption performance of various gases, repeatability (for example, the maximum number of repetitions in which the structure of MOF is not destructed even when water or gas is adsorbed or desorbed), and mechanical properties in MOF.

The configuration of the first model 121 and the second model 122 is not limited. The first model 121 and the second model 122 may be a learning model constructed by other learning algorithms such as Lasso regression, non-linear regression, a support vector machine, random forests, and a neural network.

The first model 121 and the second model 122 are not limited to a model created by the information processing device 1, but a model created by an external device connected with the information processing device 1 and capable of communicating with it may be acquired by the information processing device 1 and stored in the storage unit 12.

Next, a method for extracting MOF in this embodiment will be described. Hereinafter, an example of extracting MOF satisfying a predetermined water adsorption performance among the existing MOFs will be described.

In the extraction of MOF, the types of first parameter and second parameter to be an explanatory variable, and the type of adsorption-desorption characteristic to be an object variable are set in advance. In the following description, the specific surface area, the pore volume, the crystal density, and the pore diameter are used as the first parameter, and the porosity is used as the second parameter. As the adsorption-desorption characteristic, the first water adsorption performance and the second water adsorption performance are used. Note that, in this embodiment, two types of adsorption-desorption characteristics are evaluated, but the adsorption-desorption characteristic to be an evaluation target may be one type, or three or more types.

The information processing device 1 acquires MOF data including various parameters relevant to a plurality of existing MOFs and the adsorption-desorption characteristic with reference to a database retaining information relevant to the existing MOF. The database may be stored in the own device, or may be stored in an external institution with the information processing device 1 and capable of communicating with it. It is preferable that the information processing device 1 widely collects the information of various MOFs using one or a plurality of databases. The information processing device 1 may extract only MOF that is known to have a predetermined characteristic (for example, the adsorption of water), among MOFs stored in the database.

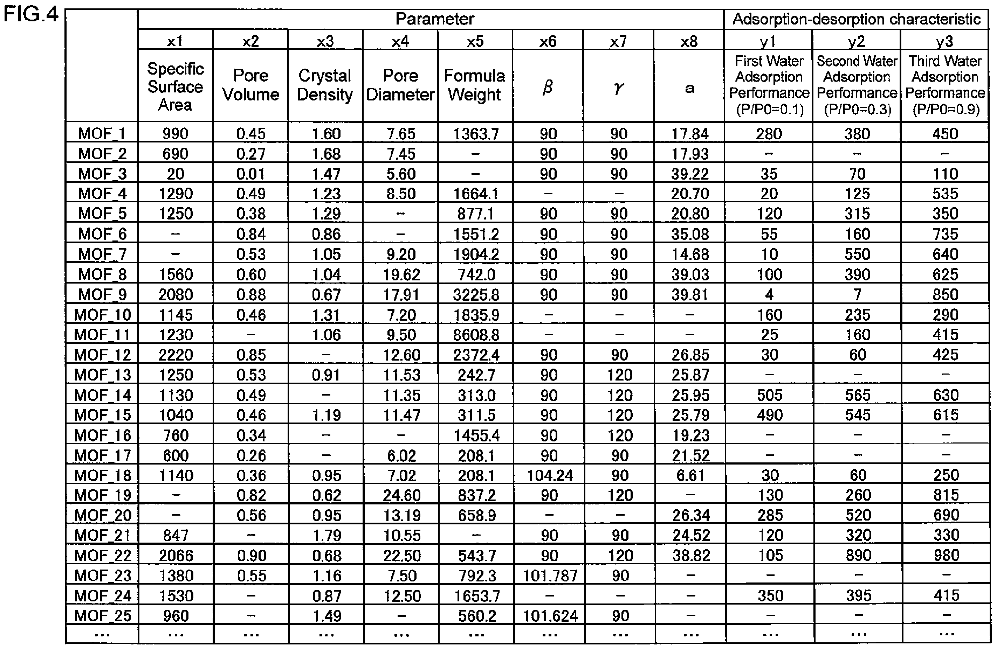

FIG. 4 is a diagram illustrating a content example of the MOF data. The MOF data, for example, includes a record in which the plurality of parameters and the information of a plurality of adsorption-desorption characteristics are linked using identification information (for example, the ID, the name, or the like of MOF) for identifying MOF as a key.

The parameters included in the MOF data include not only the first parameter (in the example illustrated in FIG. 4, x1 indicating the specific surface area, x2 indicating the pore volume, x3 indicating the crystal density, and x4 indicating the pore diameter), but also a plurality of other parameters (in the example illustrated in FIG. 4, x5 indicating the formula weight, x6 indicating a lattice constant (an interaxis angle) β, x7 indicating a lattice constant (an interaxis angle) γ, and x8 indicating a lattice constant (the length of the axis) a). The parameters other than the first parameter may include various parameters that are generally observed as parameters relevant to MOF.

The adsorption-desorption characteristic included in the MOF data includes the water adsorption performance to be the evaluation target (in the example illustrated in FIG. 4, y1 indicating the first water adsorption performance, and y2 indicating the second water adsorption performance), and a water adsorption performance other than the evaluation target (in the example illustrated in FIG. 4, y3 indicating a third water adsorption performance). Note that, the adsorption-desorption characteristic of the MOF data may include at least the water adsorption performance to be the evaluation target.

As illustrated in FIG. 4, the record of each MOF included in the MOF data includes a record in which all the data is recorded, and a record in which a part of the data is missed.

First, MOF not including a missing value in each of the parameters x1 to x8 and the water adsorption performances y1 and y2 is specified among the plurality of MOFs. In this case, only a predetermined number of other parameters including at least the first parameter may be a target for determining the presence or absence of the missing value. Examples of the other predetermined parameters include a parameter capable of affecting the adsorption-desorption characteristic. The information processing device 1 acquires the parameters x1 to x8 and the adsorption-desorption characteristics y1 and y2 in a plurality of specified MOFs.

On the basis of a correlative relationship between each of the acquired parameters x1 to x8 and each of the adsorption-desorption characteristics y1 and y2, a parameter correlated with the adsorption-desorption characteristics y1 and y2 (hereinafter, also referred to as a focused parameter) is specified among the each of the parameters x1 to x8. Specifically, a parameter is specified in which a correlation coefficient calculated on the basis of each of the parameters x1 to x8 and the first water adsorption performance y1 is a predetermined value or more. Similarly, a parameter is specified in which a correlation coefficient calculated on the basis of each of the parameters x1 to x8 and the second water adsorption performance y2 is a predetermined value or more. Alternatively, a predetermined number of parameters may be specified in descending order of the correlation coefficient.

By comprehensively evaluating the correlation coefficient of the specified parameter, one focused parameter strongly correlated with both of the first water adsorption performance y1 and the second water adsorption performance y2 is specified among the specified parameters. For example, the information processing device 1 may select a parameter with the highest total value of the correlation coefficient between the first water adsorption performance y1 and the second water adsorption performance y2, as the focused parameter.

The correlation coefficient, for example, can be calculated by using a calculation formula (correlation coefficient =covariance of value of first data included in plurality of data pieces and value of second data other than first data included in plurality of data pieces/(standard deviation of value of first data × standard deviation of value of second data)).

The focused parameter is not limited to a parameter determined by the information processing device 1, and may be acquired by receiving the selection from the user such as a person skilled in the art. The information processing device 1 outputs the correlation coefficient calculated by the processing described above through the display unit 14. In this case, the information processing device 1 may output the correlation coefficient by visualization, for example, using a heat map.

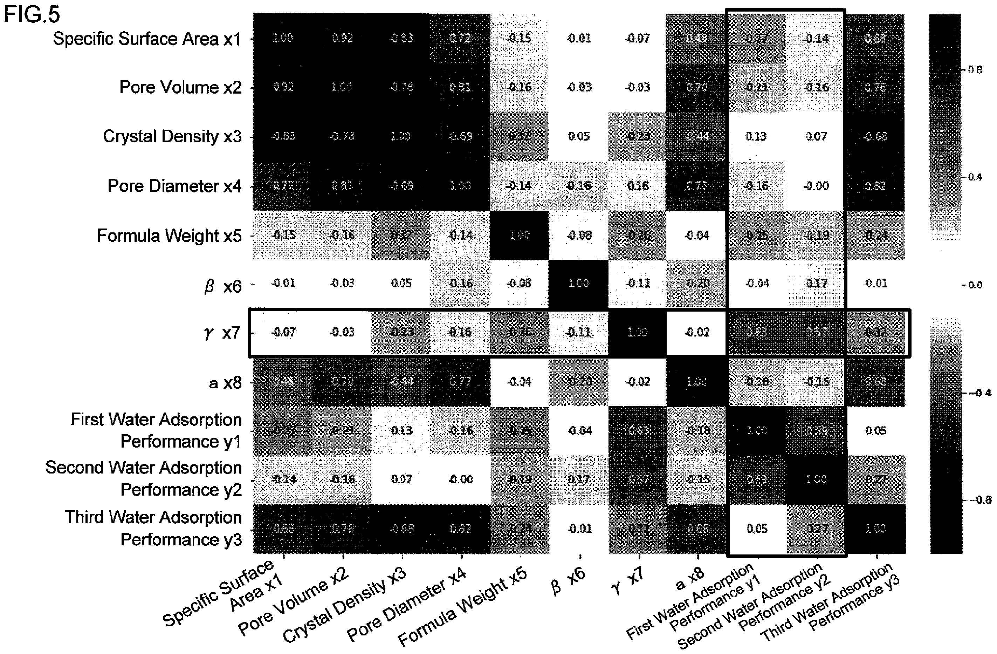

FIG. 5 is a diagram illustrating an example of a heat map indicating a correlative relationship. In the example illustrated in FIG. 5, the heat map is a matrix chart in which both of a vertical axis and a horizontal axis are the types of parameters $x_1$ to x8 and adsorption-desorption characteristics y1 to y3. In each region in the matrix chart, a correlation coefficient between the corresponding parameter and adsorption-desorption characteristic is displayed, and coloring according to the correlation coefficient is performed. A coloring method is not limited, and for example, a region having a positive correlation is colored with red, and a region having a negative correlation is colored with blue, and the concentration value of each region is changed such that the concentration value increases as the numerical value (the absolute value) of the correlation coefficient increases.

The user can clearly recognize a parameter having a strong correlation with the first water adsorption performance y1 and the second water adsorption performance y2 by the heat map. In the example illustrated in FIG. 5, it is illustrated that x7 indicating the lattice constant γ has the highest correlation with both of the first water adsorption performance y1 and the second water adsorption performance y2. Hereinafter, x7 indicating the lattice constant γ is designated as the focused parameter. Note that, two or more types of focused parameters may be specified.

Next, an extraction condition for the focused parameter x7 is determined. The extraction condition may be suitably set on the basis of the first water adsorption performance y1 and the second water adsorption performance y2 of each of the MOFs included in the MOF data, and the value of the focused parameter x7. In this embodiment, the threshold value (the lower limit value) of the focused parameter x7 is set on the basis of the focused parameter x7 of MOF of which the first water adsorption performance y1 and the second water adsorption performance y2 are a predetermined value or more. As an example, extraction condition is $\gamma \geq 120$.

The information processing device 1 again refers to the information of the plurality of existing MOFs stored in the database of the own device or the external institution, and extracts MOF satisfying the extraction condition for the focused parameter x7, that is, MOF of which $\gamma$ is 120° or more, among all MOFs. Accordingly, primary extraction using the focused parameter is completed.

Next, the second parameter in each MOF subjected to the primary extraction is acquired by using the second model 122. The information processing device 1 estimates the porosity that is the second parameter by inputting the first parameters x1 to x4 of each MOF, which are obtained by referring to the database, to the second model 122. The information processing device 1 may exclude MOF of which the first parameters x1 to x4 are unavailable when acquiring the second parameter.

The porosity is not limited to the porosity acquired by using the second model 122, and for example, may be acquired on the basis of the description in theses, technical books, or the like. Note that, for MOF of which the porosity is known in advance, estimation processing using the second model 122 is not required.

An extraction condition for the porosity is determined on the basis of the obtained porosity of each MOF. The extraction condition may be suitably set on the basis of the first water adsorption performance y1 and the second water adsorption performance y2 of each MOF, and the value of the porosity. In this case, it is preferable to set the extraction condition by selecting only MOF of which the first water adsorption performance y1 and the second water adsorption performance y2 are known. In this embodiment, the threshold value (the lower limit value) of the porosity is set on the basis of the porosity of MOF of which the known first water adsorption performance y1 and second water adsorption performance y2 are a predetermined value or more. As the lower limit value of the porosity, for example, the minimum value of the porosity in MOF of which the first water adsorption performance y1 and the second water adsorption performance y2 are a predetermined value or more may be used, or a value may be used in which a predetermined margin is added to the minimum value.

The information processing device 1 extracts MOF satisfying the extraction condition for the porosity that is the second parameter, that is, MOF having porosity $\geq$ predetermined threshold value (lower limit value), among MOFs subjected to the primary extraction. Accordingly, secondary extraction using the second parameter is completed.

For each MOF subjected to the secondary extraction, the first water adsorption performance y1 and the second water adsorption performance y2 are evaluated by using the first model 121. The information processing device 1 acquires the first water adsorption performance y1 and the second water adsorption performance y2 by inputting the first parameters x1 to x4 and the second parameter of each MOF subjected to the secondary extraction to the first model 121.

An extraction condition for the first water adsorption performance y1 and the second water adsorption performance y2 is determined on the basis of the obtained first water adsorption performance y1 and second water adsorption performance y2 of each MOF. The extraction condition may be suitably set according to the adsorption-desorption characteristic required for MOF. As an example, the extraction condition is first water adsorption performance y1 $\geq$ predetermined threshold value (lower limit value), second water adsorption performance y2 $\geq$ predetermined threshold value (lower limit value).

The information processing device 1 extracts MOF satisfying the extraction condition for the first water adsorption performance y1 and the second water adsorption performance y2, that is, MOF of which the first water adsorption performance y1 and second water adsorption performance y2 are each lower limit value or more, among MOFs subjected to the secondary extraction. Accordingly, tertiary extraction using the adsorption-desorption characteristic is completed.

The extraction condition for the first water adsorption performance y1 and the second water adsorption performance y2 is not limited to the lower limit value sorting, and for example, may be a condition for extracting, on the basis of the numerical value of the first water adsorption performance y1 and the second water adsorption performance y2, a predetermined number of MOFs in descending order of the numerical value.

The information processing device 1 outputs the result of the tertiary extraction through the display unit 14. The information processing device 1, for example, may output the result of the tertiary extraction by visualization using a heat map.

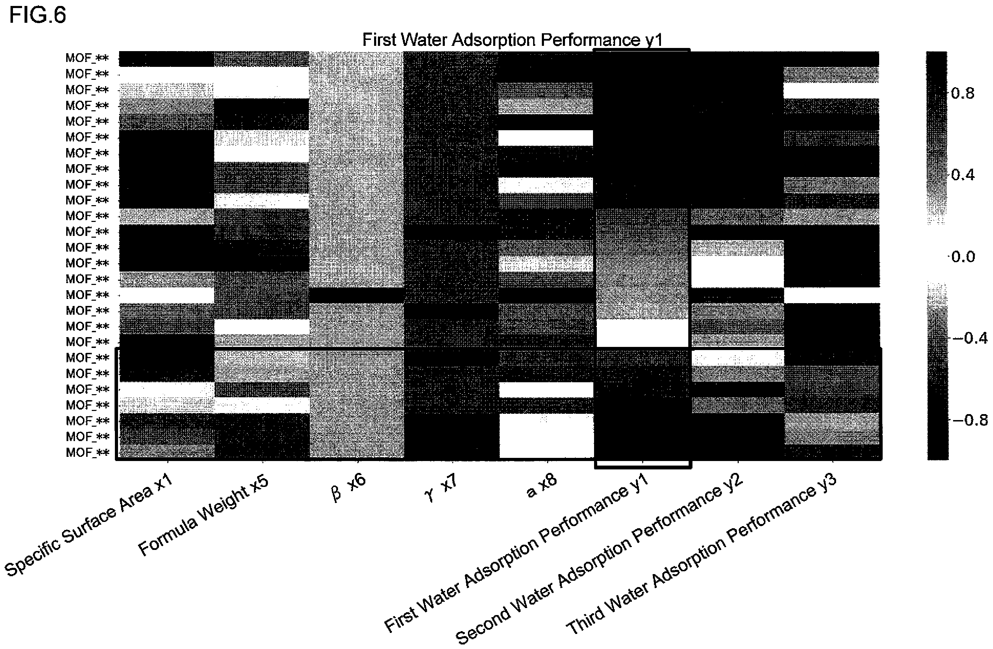

FIG. 6 is a diagram illustrating an example of a heat map indicating an extraction result. FIG. 6 illustrates an example of the heat map indicating the extraction result on the basis of the first water adsorption performance y1. In the example illustrated in FIG. 6, the heat map is a diagram in which a vertical axis is the identification information of MOF, and a horizontal axis is the first water adsorption performance y1. In FIG. 6, as an item for the horizontal axis, the first parameter, the second parameter, the focused parameter, and other adsorption-desorption characteristics are further included. Other items for the horizontal axis are not essential, but the relationship with the other items can be efficiently grasped by displaying the other items in parallel.

MOF indicated on the vertical axis is arranged according to the value of the first water adsorption performance y1. In the example illustrated in FIG. 6, MOF with the smallest first water adsorption performance y1 is disposed on at the top. In each region of the drawing, coloring according to the first water adsorption performance y1 is performed. Note that, the first water adsorption performance y1 is subjected in advance to normalization processing in which the maximum value of the first water adsorption performance y1 in all MOFs to be a display target is set to 1, and the minimum value thereof is set to -1. The information processing device 1, for example, colors a region in which the first water adsorption performance y1 is a positive value with red, and a region in which the first water adsorption performance y1 is a negative value with blue, and the concentration value of each region is changed such that the concentration value increases as the numerical value (the absolute value) of the first water adsorption performance y1 increases.

The information processing device 1, similarly, may create and output the heat map indicating the extraction result on the basis of the second adsorption performance y2. The user can clearly recognize an evaluation result of the first water adsorption performance y1 and the second water adsorption performance y2 for each MOF by the heat map.

The user finally determines MOF to be a production candidate, on the basis of the result of the tertiary extraction, with reference to the synthesis difficulty of each of the extracted MOFs, the obtainment difficulty of the organic ligand, or the like.

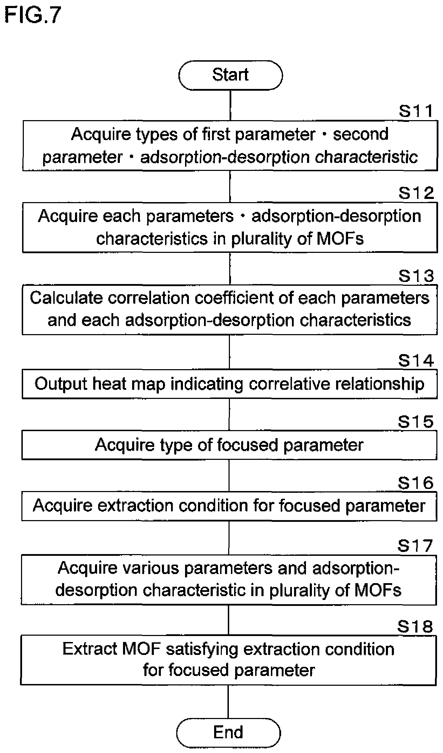

FIG. 7 is a flowchart illustrating an example of a processing procedure relevant to the primary extraction. The processing in each of the following flowcharts may be executed by the control unit 11 according to the program 1P stored in the storage unit 12 of the information processing device 1, may be attained by a dedicated hardware circuit (for example, FPGA or ASIC) provided in the control unit 11, or may be attained by a combination thereof.

The control unit 11 of the information processing device 1 acquires the types of first parameters x1 to x4, second parameter, and adsorption-desorption characteristics y1 and y2 used for the extraction processing (step S11). The control unit 11, for example, may acquire the type by receiving the input from the user manipulating the manipulation unit 15, or may acquire the type by receiving information transmitted from an external device connected such that communication is available.

The control unit 11 acquires the MOF data including each of the parameters x1 to x8 and the adsorption-desorption characteristics y1 and y2 in the plurality of existing MOFs, with reference to the database of the own device or the external institution (step S12). The control unit 11 extracts only MOF in which there is no missing value in each of the parameters x1 to x8 and the adsorption-desorption characteristics y1 and y2, among the plurality of MOFs in the acquired MOF data.

The control unit 11 calculates correlation coefficient of each of the parameters x1 to x8 and each of the adsorption-desorption characteristics y1 and y2 in the extracted MOF by using a predetermined calculation formula (step S13). The control unit 11 creates the heat map (refer to FIG. 5) indicating the correlative relationship between each of the parameters x1 to x8 and each of the adsorption-desorption characteristics y1 and y2, on the basis of the calculated correlation coefficient, and outputs the created heat map (step S14). The control unit 11 changes and displays the color of each region in the heat map, and the concentration value thereof, according to the correlation coefficient of each of the parameters x1 to x8 and each of the adsorption-desorption characteristics y1 and y2.

The control unit 11 acquires the type of focused parameter correlated with the adsorption-desorption characteristics y1 and y2, among the each of the parameters x1 to x8 (step S15). Further, the control unit 11 acquires the extraction condition for the focused parameter (step S16). The control unit 11 may acquire the focused parameter and the extraction condition by receiving the input from the user manipulating the manipulation unit 15. The control unit 11 may specify the focused parameter and the extraction condition according to a predetermined rule.

The control unit 11 acquires various parameters and the adsorption-desorption characteristic in the plurality of existing MOFs with reference to the database of the own device or the external institution (step S17). The control unit 11 extracts MOF satisfying the extraction condition for the focused parameter, among all MOFs for which various parameters and the adsorption-desorption characteristic are acquired (step S18). As an example, the control unit 11 extracts MOF of which the lattice constant $\gamma$ that is the focused parameter is 120° or more. By the processing described above, the primary extraction is ended.

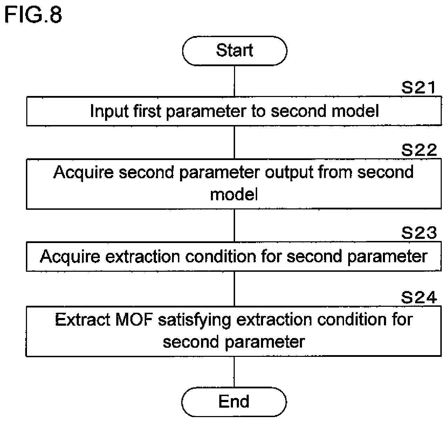

FIG. 8 is a flowchart illustrating an example of a processing procedure relevant to the secondary extraction.

The control unit 11 of the information processing device 1 inputs the first parameters x1 to x4 in each MOF extracted in step S18 of FIG. 7 to the second model 122 (step S21). The control unit 11 acquires the second parameter (for example, the porosity) output from the second model 122 (step S22).

The control unit 11 acquires the extraction condition for the second parameter (step S23). The extraction condition for the second parameter is set on the basis of the value of the porosity in MOF of which the adsorption-desorption characteristics y1 and y2 are known. The control unit 11, for example, may acquire the extraction condition by receiving the input from the user manipulating the manipulation unit 15, or may specify the extraction condition according to a predetermined rule.

The control unit 11 extracts MOF satisfying the extraction condition for the second parameter, among all MOFs subjected to the primary extraction (step S24). As an example, the control unit 11 extracts MOF of which the porosity is a lower limit value or more. By the processing described above, the secondary extraction is ended.

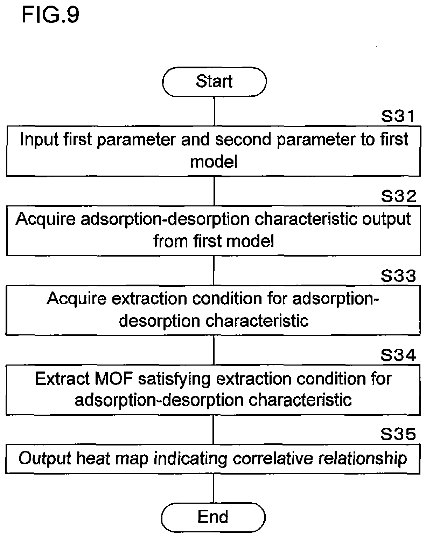

FIG. 9 is a flowchart illustrating an example of a processing procedure relevant to the tertiary extraction.

The control unit 11 of the information processing device 1 inputs the first parameters x1 to x4 in each MOF extracted in step S24 of FIG. 8, and the second parameter acquired in step S22 to the first model 121 (step S31). The control unit 11 acquires the adsorption-desorption characteristics y1 and y2 output from the first model 121 (step S32). The control unit 11 may acquire each of the adsorption-desorption characteristics y1 and y2 output from each of a plurality of first models 121 by inputting the first parameters x1 to x4 and the second parameter to each of the plurality of first models 121 outputting each of the adsorption-desorption characteristics y1 and y2.

The control unit 11 acquires the extraction condition for each of the adsorption-desorption characteristics y1 and y2 (step S33). The control unit 11, for example, may acquire the extraction condition by receiving the input from the user manipulating the manipulation unit 15, or may specify the extraction condition, according to a predetermined rule.

The control unit 11 extracts MOF satisfying the extraction condition for each of the adsorption-desorption characteristics y1 and y2, among all MOFs subjected to the secondary extraction (step S34). As an example, the control unit 11 extracts MOF of which the first water adsorption performance y1 and the second water adsorption performance y2 are each a lower limit value or more. As described above, the tertiary extraction is executed.

The control unit 11 creates the heat map (refer to FIG. 6) indicating the extraction result, and outputs the created heat map (step S35). The control unit 11 arranges MOFs according to each of the adsorption-desorption characteristics y1 and y2 in each of the extracted MOFs, and changes and displays the color of each region in the heat map, and the concentration value thereof, according to each of the adsorption-desorption characteristics y1 and y2.

In the processing described above, the control unit 11 may execute each processing piece by including the third water adsorption performance y3 as the adsorption-desorption characteristic. The third water adsorption performance y3 is obtained by the same processing as that of the first water adsorption performance y1 and the second water adsorption performance y2.

Next, a method for producing MOF according to this embodiment, to which the information processing method according to this embodiment is applied, will be described.

The method for producing MOF according to this embodiment includes a step of acquiring the plurality of parameters in MOF to be the candidate, a step of outputting the adsorption-desorption characteristic by inputting the plurality of acquired parameters to the first model 121 trained to output an adsorption-desorption characteristic relevant to the adsorption or the desorption in MOF when a plurality of parameters in MOF are input, a step of specifying MOF of which the output adsorption-desorption characteristic satisfies a predetermined condition, and a step of obtaining the specified MOF.

The step of acquiring the plurality of parameters in MOF to be the candidate, and the step of outputting the adsorption-desorption characteristic by inputting the plurality of acquired parameters to the first model 121 can be performed in the same manner as the information processing method described above.

The step of specifying MOF of which the output adsorption-desorption characteristic satisfies the predetermined condition may include a step of specifying MOF on the basis of the estimation value of the adsorption-desorption characteristic, and a step of specifying MOF on the basis of the actually measured value of the adsorption-desorption characteristic.

In the step of specifying MOF on the basis of the estimation value of the adsorption-desorption characteristic, MOF of which the adsorption-desorption characteristic output from the first model 121 satisfies the predetermined condition is extracted by performing third extraction as with the information processing method described above.

In the step of specifying MOF on the basis of the actually measured value of the adsorption-desorption characteristic, the actually measured value of the adsorption-desorption characteristic is acquired by actually measuring (observing) the adsorption-desorption characteristic in the extracted MOF. Whether the acquired actually measured value of the adsorption-desorption characteristic satisfies the condition of the adsorption-desorption characteristic to be the production target is determined to specify one or a predetermined number of MOFs to be the production target.

In the step of obtaining the specified MOF, for example, MOF can be obtained by mixing a metal ion source and an organic ligand or a salt thereof using a hydrothermal synthesis method or a solvothermal synthesis method.

In this embodiment, MOF obtained by the information processing method can be provided. MOF obtained by the information processing method is MOF to which the information processing method is applied, and specifically, is MOF of which the adsorption-desorption characteristic relevant to the adsorption or the desorption is output by the information processing method.

In this embodiment, a complex containing MOF obtained by the information processing method can be provided. The complex containing MOF obtained by the information processing method is a complex containing MOF to which the information processing method is applied, and specifically, is a complex containing MOF of which the adsorption-desorption characteristic relevant to the adsorption or the desorption is output by the information processing method. The complex, for example, can be obtained by mixing a fiber, cellulose, glass, a resin, a metal, and the like with MOF described above, or attaching MOF described above.

According to this embodiment, it is possible to easily and accurately estimate the adsorption-desorption characteristic of MOF by using the first model 121 and the second model 122. By using the obtained adsorption-desorption characteristic, it is possible to efficiently extract MOF with an excellent adsorption-desorption characteristic.

The parameter input to the first model 121 used for the estimation of the adsorption-desorption characteristic includes the porosity. The porosity may have a correlation with the water adsorption performance, but is a parameter difficult to observe, and thus, is difficult to use as an input element for optimizing the adsorption-desorption characteristic. In this embodiment, by using the second model 122, it is possible to efficiently estimate such a parameter difficult to observe. By evaluating the adsorption-desorption characteristic including the obtained parameter, it is possible to more efficiently extract MOF of which the adsorption-desorption characteristic is optimized.

According to this embodiment, by focusing on the relationship between the known adsorption-desorption characteristic in MOF and the focused parameter or the porosity, it is possible to efficiently specify a suitable extraction condition.

The disclosed embodiments as described above are illustrative in all respects and are not restrictive. The scope of the present invention is indicated by the claims, and includes the meaning equivalent to the claims and all modifications within the claims. In addition, at least some of the embodiments described above may be arbitrarily combined.

The sequences represented in the embodiments described above are not limited, and the order of each processing procedure may be changed, or a plurality of processing pieces may be executed in parallel, unless there is a contradiction. The subject of each processing piece is not limited, and the processing of each device may be executed by another device, unless there is a contradiction.

Reference Signs List

| 1   | information processing device |
|-----|-------------------------------|
| 11  | control unit                  |
| 12  | storage unit                  |
| 13  | communication unit            |
| 14  | display unit                  |
| 15  | manipulation unit             |
| 1P  | program                       |
| 121 | first model                   |
| 122 | second model                  |
| 1A  | recording medium              |

**Claims**

1. An information processing method, comprising:

    acquiring a plurality of parameters in a metal organic framework; and
    outputting an adsorption-desorption characteristic by inputting the plurality of acquired parameters to a first model trained to output an adsorption-desorption characteristic relevant to adsorption or desorption in a metal organic framework when a plurality of parameters in the metal organic framework are input.

2. The information processing method according to claim 1, wherein

    the parameters include a first parameter and a second parameter different from the first parameter, and
    the method comprises outputting the second parameter by inputting the acquired first parameter to a second model trained to output a second parameter when a first parameter in a metal organic framework is input.

3. The information processing method according to claim 2, further comprising
inputting the first parameter in the metal organic framework, and the second parameter obtained by using the first parameter and the second model to the first model.

4. The information processing method according to claim 2 or 3,
wherein the second parameter is a porosity.

5. The information processing method according to any one of claims 1 to 4, further comprising
extracting a metal organic framework of which the adsorption-desorption characteristic satisfies a predetermined condition on the basis of the adsorption-desorption characteristic output by the first model.

6. The information processing method according to any one of claims 1 to 5,
wherein the first model is trained to output adsorption performance of water in a metal organic framework when at least one selected from a specific surface area, a pore volume, a crystal density, and a pore diameter in a metal organic framework, and a porosity are input.

7. The information processing method according to any one of claims 1 to 6, further comprising
specifying a parameter correlated with the adsorption-desorption characteristic among the plurality of parameters on the basis of a correlative relationship between the plurality of parameters in the metal organic framework and the adsorption-desorption characteristic relevant to the adsorption or the desorption in the metal organic framework.

8. The information processing method according to claim 7, further comprising

extracting a metal organic framework of which the specified parameter correlated with the adsorption-desorption characteristic satisfies a predetermined condition, and
outputting the adsorption-desorption characteristic using the first model to the extracted metal organic framework.

9. A program for causing a computer to execute processing of:

acquiring a plurality of parameters in a metal organic framework; and
outputting an adsorption-desorption characteristic by inputting the plurality of acquired parameters to a first model trained to output an adsorption-desorption characteristic relevant to adsorption or desorption in a metal organic framework when a plurality of parameters in the metal organic framework are input.

10. An information processing device, comprising a control unit executing processing of:

acquiring a plurality of parameters in a metal organic framework; and
outputting an adsorption-desorption characteristic by inputting the plurality of acquired parameters to a first model trained to output an adsorption-desorption characteristic relevant to adsorption or desorption in a metal organic framework when a plurality of parameters in the metal organic framework are input.

11. A method for creating a model, comprising:

acquiring training data including a plurality of parameters in a metal organic framework, and an adsorption-desorption characteristic relevant to adsorption or desorption in the metal organic framework; and
creating a model trained to output an adsorption-desorption characteristic relevant to adsorption or desorption in a metal organic framework when a plurality of parameters in the metal organic framework are input, on the basis of the training data.

12. A method for producing a metal organic framework, comprising:

a step of acquiring a plurality of parameters in a metal organic framework to be a candidate;
a step of outputting an adsorption-desorption characteristic by inputting the plurality of acquired parameters to a first model trained to output an adsorption-desorption characteristic relevant to adsorption or desorption in a metal organic framework when a plurality of parameters in the metal organic framework are input;
a step of specifying a metal organic framework of which the output adsorption-desorption characteristic satisfies a predetermined condition; and
a step of obtaining the specified metal organic framework.

13. A metal organic framework obtained by the information processing method according to any one of claims 1 to 8.

14. A complex containing a metal organic framework obtained by the information processing method according to any one of claims 1 to 8.

FIG.1

Information Processing Device — 1

Control Unit — 11

Communication Unit — 13

Display Unit — 14

Manipulation Unit — 15

Storage Unit — 12

Program — 1P

First Model — 121

Second Model — 122

1A

FIG.2

Input Data                                                          Output Data

•First Parameter
    Specific Surface Area
    Pore Volume                            ⌐121                      Water
    Crystal Density            ⟶    | First Model |    ⟶    Adsorption
    Pore Diameter                                                   Performance
•Second Parameter
    Porosity

# FIG.3

Input Data                                                                    Output Data

First Parameter
   Specific Surface Area     →     ┌──────────┐    →     Second Parameter
   Pore Volume

_122

Second
Model

Second Parameter
Porosity

First Parameter
   Specific Surface Area
   Pore Volume
   Crystal Density
   Pore Diameter

**FIG.4**

| | Parameter | | | | | | | | Adsorption-desorption characteristic | | |
|---|---|---|---|---|---|---|---|---|---|---|---|
| | x1 | x2 | x3 | x4 | x5 | x6 | x7 | x8 | y1 | y2 | y3 |
| | Specific Surface Area | Pore Volume | Crystal Density | Pore Diameter | Formula Weight | $\beta$ | $\gamma$ | a | First Water Adsorption Performance (P/P0=0.1) | Second Water Adsorption Performance (P/P0=0.3) | Third Water Adsorption Performance (P/P0=0.9) |
| MOF_1 | 990 | 0.45 | 1.60 | 7.65 | 1363.7 | 90 | 90 | 17.84 | 280 | 380 | 450 |
| MOF_2 | 690 | 0.27 | 1.68 | 7.45 | – | 90 | 90 | 17.93 | – | – | – |
| MOF_3 | 20 | 0.01 | 1.47 | 5.60 | – | 90 | 90 | 39.22 | 35 | 70 | 110 |
| MOF_4 | 1290 | 0.49 | 1.23 | 8.50 | 1664.1 | – | – | 20.70 | 20 | 125 | 535 |
| MOF_5 | 1250 | 0.38 | 1.29 | – | 877.1 | 90 | 90 | 20.80 | 120 | 315 | 350 |
| MOF_6 | – | 0.84 | 0.86 | – | 1551.2 | 90 | 90 | 35.08 | 55 | 160 | 735 |
| MOF_7 | – | 0.53 | 1.05 | 9.20 | 1904.2 | 90 | 90 | 14.68 | 10 | 550 | 640 |
| MOF_8 | 1560 | 0.60 | 1.04 | 19.62 | 742.0 | 90 | 90 | 39.03 | 100 | 390 | 625 |
| MOF_9 | 2080 | 0.88 | 0.67 | 17.91 | 3225.8 | 90 | 90 | 39.81 | 4 | 7 | 850 |
| MOF_10 | 1145 | 0.46 | 1.31 | 7.20 | 1835.9 | – | – | – | 160 | 235 | 290 |
| MOF_11 | 1230 | – | 1.06 | 9.50 | 8608.8 | – | – | – | 25 | 160 | 415 |
| MOF_12 | 2220 | 0.85 | – | 12.60 | 2372.4 | 90 | 90 | 26.85 | 30 | 60 | 425 |
| MOF_13 | 1250 | 0.53 | 0.91 | 11.53 | 242.7 | 90 | 120 | 25.87 | – | – | – |
| MOF_14 | 1130 | 0.49 | – | 11.35 | 313.0 | 90 | 120 | 25.95 | 505 | 565 | 630 |
| MOF_15 | 1040 | 0.46 | 1.19 | 11.47 | 311.5 | 90 | 120 | 25.79 | 490 | 545 | 615 |
| MOF_16 | 760 | 0.34 | – | – | 1455.4 | 90 | 120 | 19.23 | – | – | – |
| MOF_17 | 600 | 0.26 | – | 6.02 | 208.1 | 90 | 90 | 21.52 | – | – | – |
| MOF_18 | 1140 | 0.36 | 0.95 | 7.02 | 208.1 | 104.24 | 90 | 6.61 | 30 | 60 | 250 |
| MOF_19 | – | 0.82 | 0.62 | 24.60 | 837.2 | 90 | 120 | – | 130 | 260 | 815 |
| MOF_20 | – | 0.56 | 0.95 | 13.19 | 658.9 | – | – | 26.34 | 285 | 520 | 690 |
| MOF_21 | 847 | – | 1.79 | 10.55 | – | 90 | 90 | 24.52 | 120 | 320 | 330 |
| MOF_22 | 2066 | 0.90 | 0.68 | 22.50 | 543.7 | 90 | 120 | 38.82 | 105 | 890 | 980 |
| MOF_23 | 1380 | 0.55 | 1.16 | 7.50 | 792.3 | 101.787 | 90 | – | – | – | – |
| MOF_24 | 1530 | – | 0.87 | 12.50 | 1653.7 | – | – | – | 350 | 395 | 415 |
| MOF_25 | 960 | – | 1.49 | – | 560.2 | 101.624 | 90 | – | – | – | – |
| ... | ... | ... | ... | ... | ... | ... | ... | ... | ... | ... | ... |

EP 4 475 132 A1

FIG.5

|  | Specific Surface Area x1 | Pore Volume x2 | Crystal Density x3 | Pore Diameter x4 | Formula Weight x5 | β x6 | γ x7 | a x8 | First Water Adsorption Performance y1 | Second Water Adsorption Performance y2 | Third Water Adsorption Performance y3 |
|---|---|---|---|---|---|---|---|---|---|---|---|
| Specific Surface Area x1 | 1.00 | 0.92 | -0.83 | 0.72 | -0.15 | -0.01 | -0.07 | 0.48 | 0.27 | -0.14 | 0.68 |
| Pore Volume x2 | 0.92 | 1.00 | -0.78 | 0.81 | -0.16 | -0.03 | -0.03 | 0.70 | 0.21 | -0.16 | 0.76 |
| Crystal Density x3 | -0.83 | -0.78 | 1.00 | -0.69 | 0.32 | 0.05 | -0.23 | -0.44 | 0.13 | 0.07 | -0.68 |
| Pore Diameter x4 | 0.72 | 0.81 | -0.69 | 1.00 | -0.14 | -0.16 | 0.16 | 0.77 | -0.16 | -0.00 | 0.82 |
| Formula Weight x5 | -0.15 | -0.16 | 0.32 | -0.14 | 1.00 | -0.08 | -0.26 | -0.04 | 0.25 | -0.19 | 0.24 |
| β x6 | -0.01 | -0.03 | 0.05 | -0.16 | -0.08 | 1.00 | -0.11 | -0.20 | -0.04 | 0.17 | -0.01 |
| γ x7 | -0.07 | -0.03 | -0.23 | 0.16 | -0.26 | -0.11 | 1.00 | -0.02 | 0.18 | 0.57 | 0.32 |
| a x8 | 0.48 | 0.70 | -0.44 | 0.77 | -0.04 | 0.20 | -0.02 | 1.00 | -0.18 | -0.15 | 0.68 |
| First Water Adsorption Performance y1 | 0.27 | 0.21 | 0.13 | -0.16 | 0.25 | -0.04 | 0.18 | -0.18 | 1.00 | 0.59 | 0.05 |
| Second Water Adsorption Performance y2 | -0.14 | -0.16 | 0.07 | -0.00 | -0.19 | 0.17 | 0.57 | -0.15 | 0.59 | 1.00 | 0.27 |
| Third Water Adsorption Performance y3 | 0.68 | 0.76 | -0.68 | 0.82 | 0.24 | -0.01 | 0.32 | 0.68 | 0.05 | 0.27 | 1.00 |

FIG.6

First Water Adsorption Performance y1

Specific Surface Area x1 | Formula Weight x5 | β x6 | γ x7 | α x8 | First Water Adsorption Performance y1 | Second Water Adsorption Performance y2 | Third Water Adsorption Performance y3

# FIG.7

```
                    ( Start )
                        |
                        |                        S11
   ┌────────────────────┴─────────────────────┐
   │ Acquire types of first parameter · second │
   │ parameter · adsorption-desorption characteristic │
   └────────────────────┬─────────────────────┘
                        |                        S12
   ┌────────────────────┴─────────────────────┐
   │ Acquire each parameters · adsorption-desorption │
   │      characteristics in plurality of MOFs  │
   └────────────────────┬─────────────────────┘
                        |                        S13
   ┌────────────────────┴─────────────────────┐
   │ Calculate correlation coefficient of each parameters │
   │   and each adsorption-desorption characteristics │
   └────────────────────┬─────────────────────┘
                        |                        S14
   ┌────────────────────┴─────────────────────┐
   │ Output heat map indicating correlative relationship │
   └────────────────────┬─────────────────────┘
                        |                        S15
   ┌────────────────────┴─────────────────────┐
   │      Acquire type of focused parameter     │
   └────────────────────┬─────────────────────┘
                        |                        S16
   ┌────────────────────┴─────────────────────┐
   │ Acquire extraction condition for focused parameter │
   └────────────────────┬─────────────────────┘
                        |                        S17
   ┌────────────────────┴─────────────────────┐
   │ Acquire various parameters and adsorption- │
   │  desorption characteristic in plurality of MOFs │
   └────────────────────┬─────────────────────┘
                        |                        S18
   ┌────────────────────┴─────────────────────┐
   │  Extract MOF satisfying extraction condition │
   │           for focused parameter            │
   └────────────────────┬─────────────────────┘
                        |
                    ( End )
```

FIG.8

```
                    ( Start )
                        |                    S21
  +-----------------------------------------------+
  |        Input first parameter to second model   |
  +-----------------------------------------------+
                        |                    S22
  +-----------------------------------------------+
  |    Acquire second parameter output from second |
  |                     model                       |
  +-----------------------------------------------+
                        |                    S23
  +-----------------------------------------------+
  |   Acquire extraction condition for second parameter |
  +-----------------------------------------------+
                        |                    S24
  +-----------------------------------------------+
  |  Extract MOF satisfying extraction condition for |
  |                second parameter                  |
  +-----------------------------------------------+
                        |
                    (  End  )
```

# FIG.9

```
          ( Start )
                │                    S31
┌───────────────┴───────────────────┐
│ Input first parameter and second parameter to first │
│                 model              │
└───────────────┬───────────────────┘
                │                    S32
┌───────────────┴───────────────────┐
│ Acquire adsorption-desorption characteristic output │
│              from first model      │
└───────────────┬───────────────────┘
                │                    S33
┌───────────────┴───────────────────┐
│ Acquire extraction condition for adsorption- │
│            desorption characteristic │
└───────────────┬───────────────────┘
                │                    S34
┌───────────────┴───────────────────┐
│ Extract MOF satisfying extraction condition for │
│         adsorption-desorption characteristic │
└───────────────┬───────────────────┘
                │                    S35
┌───────────────┴───────────────────┐
│ Output heat map indicating correlative relationship │
└───────────────┬───────────────────┘
                │
          ( End )
```

## INTERNATIONAL SEARCH REPORT

| International application No. |
|---|
| **PCT/JP2023/000900** |

**A. CLASSIFICATION OF SUBJECT MATTER**

*G16C 60/00*(2019.01)i
FI: G16C60/00

According to International Patent Classification (IPC) or to both national classification and IPC

**B. FIELDS SEARCHED**

Minimum documentation searched (classification system followed by classification symbols)

G16C10/00 - 99/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | YUYAMA, Shunsuke. KANEKO, Hiromasa. Correlation between the Metal and Organic Components, Structure Property, and Gas-Adsorption Capacity of Metal-Organic Frameworks. Journal of chemical information and modeling [online]. 13 December 2021, vol. 61, no. 12, pp. 5785-5792, [retrieval date: 27 January 2023], <URL:https://pubs.acs.org/doi/full/10.1021/acs.jcim.1c01205> pp. 5785-5789 | 1, 4-6, 9-14 |
| Y | pp. 5785-5789 | 7-8 |
| A | entire text, all drawings | 2-3 |
| Y | 鳴川智也, 多孔質複合材料の吸着速度の解析および機械学習を用いた平衡吸着量予測, [online]. 2019, pp. 58-59, [retrieval date: 15 February 2023], <URL:https://catalog.lib.kyushu-u.ac.jp/opac_download_md/3077404/4077404_fulltext.pdf> p. 58-59, (NARUKAWA, Tomoya), non-official translation (Analysis of adsorption rate of porous-composite materials and prediction of equilibrium adsorbed amounts using machine learning) | 7-8 |

☑ Further documents are listed in the continuation of Box C.      ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **15 February 2023** | **28 February 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

## INTERNATIONAL SEARCH REPORT

| International application No. |
| --- |
| **PCT/JP2023/000900** |

**C. DOCUMENTS CONSIDERED TO BE RELEVANT**

| Category* | Citation of document, with indication, where appropriate, of the relevant passages | Relevant to claim No. |
| --- | --- | --- |
| A | CN 111755081 A (BEIJING UNIVERSITY OF CHEMICAL TECHNOLOGY) 09 October 2020 (2020-10-09)<br>entire text, all drawings | 1-14 |

Form PCT/ISA/210 (second sheet) (January 2015)

INTERNATIONAL SEARCH REPORT
Information on patent family members

International application No.

**PCT/JP2023/000900**

| Patent document cited in search report | Publication date (day/month/year) | Patent family member(s) | Publication date (day/month/year) |
|---|---|---|---|
| CN 111755081 A | 09 October 2020 | (Family: none) | |

Form PCT/ISA/210 (patent family annex) (January 2015)

**EP 4 475 132 A1**

**Patent documents cited in the description**

- JP 2020011943 A